# NOUVEAU FASCICULE DE BREVET EUROPEEN

(11) **EP 1 262 480 B2**
(45) Date de publication et mention de la décision concernant l'opposition: **04.08.2010**
(45) Mention de la délivrance du brevet: 07.09.2005
(21) Numéro de dépôt: 02291225.7
(22) Date de dépôt: 17.05.2002
(51) Int. Cl.: C07D 263/44, C07D 269/02

(54) **Procédé de préparation des N-carboxyanhydrides d'acides aminés**
Verfahren zur Herstellung von N-Carboxyanhydriden von Aminosäuren
Process for the preparation of N-carboxyanhydrides of amino acids

(30) Priorité: 31.05.2001 FR 0107140
(43) Date de publication de la demande: 04.12.2002
(73) Titulaire: ISOCHEM, 75194 Paris Cedex 04 (FR)
(72) Inventeur: Cornille, Fabrice, 91440 Bures S/Yvette (FR); Lebon, Marc, 91150 Ormoy la Riviere (FR)
(74) Mandataire: Ahner, Francis

(56) Documents cités:
- GB-A- 1 038 913
- JP-A- 4 924 958
- US-A- 5 135 754
- SOCIETY OF POLYMER SCIENCE, JAPAN: '1.3 N-Carboxyamino Acid Anhydride', 1958, KYORITSU SHUPPAN CO.,LTD. article 'Lecture of Polymer Experimentation 11: Polycondensation and Polyaddition Reaction', pages 39 - 49
- YASUO FUJIMOTO: 'Polyamino Acid: Application and Perspective', 1974, KODANSHA LTD. article '3.2.1 Sythesis Method of NCA', pages 71 - 81

## Description

L'invention concerne un procédé amélioré de préparation des N-carboxyanhydrides à partir des amino-acides correspondants et du phosgène, du diphosgène ou du triphosgène.

Les N-carboxyanhydrides (abréviation NCA) obtenus à partir des α-, β- ou γ-amino-acides sont des composés très utiles en raison de l'activation de leur fonction acide. Ils permettent en effet la réaction de cette fonction acide avec toute entité nucléophile. Ainsi l'obtention de la fonction amide par réaction avec une fonction amine est facilitée. De ce fait, ils polymérisent facilement et sont utilisés pour former des peptides. La liaison ester par réaction avec un alcool se crée également aisément. Ils sont aussi intéressants lorsqu'on souhaite réduire une fonction acide.

Plusieurs procédés sont connus pour préparer les N-carboxyanhydrides. Un des plus courants et des plus directs est le procédé selon lequel on fait réagir un amino-acide ou son chlorhydrate avec du phosgène, du diphosgène ou du triphosgène, dans un milieu solvant.

Par exemple, la demande GB 10 38 913 décrit un procédé de synthèse de N-carboxyanhydrides, dans lequel l'a-amino acide, en suspension dans un solvant, réagit avec du phosgène puis le solvant de la réaction est distillé.

Le schéma réactionnel général avec le phosgène est le suivant : 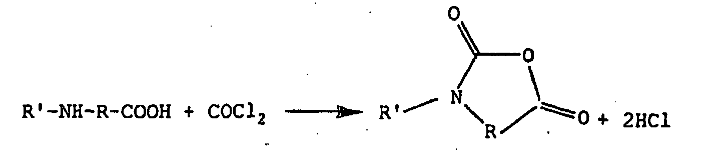 dans lequel R représente le radical principal de l'α-, β- ou γ-amino-acide et R' représente un atome d'hydrogène ou le radical du groupe amino secondaire de l'amino-acide, R' pouvant former un cycle avec R.

On constate qu'outre du N-carboxyanhydride, il se forme aussi de l'acide chlorhydrique en grande quantité, c'est-à-dire 2 moles par mole de NCA. L'acide chlorhydrique est très réactif. Sa présence dans le milieu entraîne des réactions secondaires et l'apparition de sous-produits chlorés. Ces impuretés chlorées qui restent dans les NCA produits sont tout à fait indésirables aussi bien en terme de qualité qu'en terme de rendement. En effet, elles gênent énormément la réaction de polymérisation des NCA. Pour que cette polymérisation s'effectue convenablement, il est nécessaire que la quantité de composés chlorés contenus dans les NCA monomères soit suffisamment faible. Ainsi le taux de chlore hydrolysable doit généralement être inférieur à 0,05 % en poids.

Or selon les procédés connus, lorsque la réaction est effectuée sans la présence d'un composé basique, il est difficile d'obtenir de façon répétitive un taux de chlore hydrolysable aussi faible. D'un autre côté, lorsqu'on ajoute un composé basique pour neutraliser l'acide chlorhydrique, la polymérisation des NCA, non souhaitée à ce stade, est activée et risque alors de se produire dans le milieu.

Par ailleurs, une des autres difficultés des procédés antérieurs est le choix du solvant. Il a en effet été constaté que dans des solvants tels que les esters aliphatiques comme l'acétate d'éthyle ou des solvants aprotiques apolaires comme le dichlométhane ou le toluène, la réaction de formation des NCA est généralement très lente et incomplète. Dans un solvant de la famille des éthers tel que le tétrahydrofuranne, ou le dioxanne, la réaction est plus rapide, mais ces solvants ne sont pas complètement inertes vis-à-vis du phosgène et de l'acide chlorhydrique, ce qui génère d'autres impuretés.

Il existait, par conséquent, un besoin d'améliorer le procédé existant dans lequel l'amino-acide est mis à réagir directement avec le phosgène, le diphosgène ou le triphosgène afin d'obtenir les NCA avec de meilleurs rendements et une pureté améliorée, notamment possédant un taux de chlore hydrolysable inférieur à 0,05 %. La diminution de la durée de la réaction, dans les solvants les plus inertes, était aussi fortement souhaitable.

Le procédé selon la présente invention répond à ces besoins. Ce procédé est caractérisé en ce que pour préparer les N-carboxyanhydrides, la réaction de l'α-, β- ou γ-amino-acide correspondant ou d'un de ses sels, avec le phosgène, le diphosgène et/ou le triphosgène, dans un milieu solvant, est effectuée au moins en partie sous une pression inférieure à 1000 mbar (10⁵Pa).
La présente invention a pour objet le procédé selon la revendication 1.

Ce nouveau procédé permet de résoudre les problèmes qui se posaient dans la mise en oeuvre des procédés selon l'art antérieur. Une partie de l'acide chlorhydrique est ainsi éliminée du milieu réactionnel au fur et à mesure de sa formation. Les nombreuses réactions secondaires qu'il provoquait sont supprimées et, en conséquence, l'apparition des impuretés gênantes également. De plus, le déplacement de l'équilibre réactionnel dans le sens de l'obtention du NCA souhaité est également favorisé et la cinétique de la réaction est alors accélérée.

On a aussi trouvé que dans le cas de la transformation des amino-acides dont la fonction amine est secondaire, la réalisation de la réaction sous cette pression réduite rendait inutile l'ajout, dans le milieu, d'une amine tertiaire telle que la triéthylamine ou la N-méthylmorpholine. Une telle amine était pourtant jusqu'à présent, jugée nécessaire par l'homme de métier pour effectuer la cyclisation à partir du chlorure de carbamoyle qui se forme tout d'abord dans le milieu, en tant qu'intermédiaire.

L'absence d'un réactif additionnel simplifie de plus les opérations de séparation et de récupération des produits. Le procédé est aussi pour cette raison plus économique.

Le procédé selon l'invention permet d'obtenir les N-carboxyanhydrides de la plupart des α-amino-acides et de leurs dérivés, cycliques ou non, naturels ou synthétiques, dont la fonction amine est primaire ou secondaire et notamment de tous ceux déjà connus pour réagir avec le phosgène, le diphosgène et/ou le triphosgène.

De même, il est très utile pour obtenir les N-carboxyanhydrides des β- et γ-amino-acides et de leurs dérivés, à fonction amine primaire ou secondaire. Ces composés sont en effet considérés comme difficiles à préparer selon les procédés antérieurs.

Les amino-acides qui sont utilisés comme composés de départ sont de préférence les α-, β- ou γ-amino-acides dont le ou les carbones α, β et γ le cas échéant, situés entre le groupe acide et le groupe amino réactifs, forment une chaîne hydrocarbonée alkyle, substituée ou non, pouvant être comprise en totalité ou en partie dans un radical alkyle linéaire ou ramifié, substitué ou non, et/ou dans un cycle alkyle ou hétéroalkyle, substitué ou non. Les substituants sont les groupes ou atomes que l'on trouve habituellement dans les amino-acides, tels que, par exemple, les groupes hydroxy, carboxy, mercapto, alkylthio, alkyldithio, alkyle, cycloalkyle, hétérocycloalkyle, aryle, hétéroaryle, alkyloxy, aryloxy, les atomes d'halogènes, tels que de fluor, de chlore, de brome ou d'iode, les groupes amino, guanidino ou amido substitués ou non par des groupes alkyles.

Plus précisément dans les amino-acides considérés les groupes alkyles comportent de 1 à 7 atomes de carbone et sont substitués ou non par les substituants précédemment indiqués. Les groupes aryles sont non substitués ou substitués par des substituants choisis parmi les atomes d'halogène tels que de fluor, de chlore, de brome ou d'iode et les groupes alkyle, alkoxy, aryloxy, aryle, mercapto, alkylthio, hydroxy, carboxy, amino, alkylamino, dialkylamino, nitro, trifluorométhyle. Lorsqu'ils sont présents, ces groupes substituants sont plus particulièrement en nombre de un à trois. Les groupes aryles sont en particulier les radicaux phényle on naphtyle substitués ou non.

Les groupes cycloalkyles sont constitués par des cycles ayant de 3 à 7 atomes de carbone, subtitués ou non. Les hétérocycles, qui peuvent être substitués ou non, sont des groupes cycloalkyles ou aryles qui comportent dans le cycle au moins un hétéroatome choisi parmi l'atome d'azote, d'oxygène ou de soufre.

Les substituants des groupes cycloalkyles ou hétérocycloalkyles sont choisis parmi les substituants précédemment indiqués pour les radicaux alkyles et aryles. Les substituants des groupes hétéroaryles sont choisis parmi les substituants indiqués pour les groupes aryles.

Les groupes hétéroaryles sont de préférence des groupes 2- ou 3-furanyle, 2- ou 3-thiényle, 2-, 3- ou 4-pyridinyle, 4-imidazolyle et 3-indolyle, substitués ou non.

Les amino-acides peuvent être sous leurs différentes formes et notamment lorsqu'ils possèdent un ou plusieurs carbones asymétriques sous leurs différentes formes énantiomériques, mélanges soit racémiques ou de diastéréoisomères ou encore sous forme de stéréoisomères purs.

Lorsque le radical de l'amino-acide contient des groupes fonctionnels, autres que le groupe amino et le groupe acide formant le cycle anhydride, susceptibles de réagir dans les conditions du procédé, on les masque par des groupes protecteurs, de façon connue.

Le groupe amino réactif peut être un groupe amino primaire ou secondaire. Par conséquent, l'atome d'azote peut porter un radical aliphatique, cycloaliphalique, araliphatique ou aryle, substitué ou non, comme il est habituel pour la classe des amines. En particulier, ce radical peut être substitué par les groupes précédemment indiqués comme substituants.

Le radical du groupe amino peut également former un cycle, non substitué ou substitué comme précédemment indiqué, avec le reste du radical de l'amino-acide, tel que par exemple dans la proline.

Lorsque le radical du groupe amino comporte des groupes réactifs, on les protège de façon classique.

Comme radical de ce groupe amino, on peut en particulier citer les groupes alkyles, cycloalkyles ou aralkyles non substitués ou substitués, par exemple par des groupes tels que décrits dans le brevet US n°4, 686, 295 pour les nouveaux NCA formés au moyen du phosgène, et en particulier substitués par un ou plusieurs groupes choisis parmi les groupes alkoxycarbonyles, aryloxycarbonyles et aralkyloxycarbonyles.

Comme exemples d'amino-acides, on peut citer les amino-acides les plus courants tels que glycine, alanine, valine, leucine, isoleucine, phénylalanine, sérine, thréonine, lysine, δ-hydroxylysine, arginine, ornithine, acide aspartique, asparagine, acide glutamique, glutamine, cystéine, cystine, méthionine, tyrosine, thyroxine, proline, hydroxyproline, tryptophane, l'histidine et leurs dérivés.

A la place de l'amino-acide, on peut utiliser un de ses sels comme composé de départ. Par sels de l'amino-acide, on entend les sels obtenus par réaction du groupe amino avec des acides organiques ou minéraux, tels que par exemple les sulfates, acétates, toluènesulfonates, méthanesulfonates et de préférence les halohydrates, en particulier les chlorhydrates et bromhydrates.

Les chlorhydrates sont les sels préférés.

Le procédé convient bien pour obtenir les N-carboxyanhydrides des amino-acides telles que la N-(1-éthoxy-carbonyl-3-phénylpropyl)alanine, la leucine, l'alanine, la N-trifluoroacétyl-lysine, l'ester γ-benzylique ou l'ester γ-méthylique de l'acide glutamique.

Pour la mise en oeuvre du procédé, le phosgène, le diphosgène et/ou le triphosgène peuvent être mis à réagir avec l'amino-acide pour former le cycle du N-carboxyanhydride. Le phosgène est le composé préféré.

Par rapport à l'amino-acide, un grand excès de phosgène n'est pas nécessaire. Ainsi de préférence, on ajoute environ de 1 à 3 moles de phosgène par mole d'amino-acide ou de son sel.

Le diphosgène ou le triphosgène sont ajoutés en quantité correspondante afin d'obtenir les mêmes rapports phosgène/amino-acide.

La réaction est réalisée dans un solvant aprotique et polaire. On choisit un solvant appartenant à la famille des esters aliphatiques.

Les solvants appartenant à la famille des esters ont l'avantage de ne pas réagir avec le phosgène ou l'acide chlorhydrique.

Selon l'invention, la réaction est au moins en partie effectuée à une pression inférieure ou égale à 960 mbar (96.10³Pa).

Le solvant est l'acétate d'éthyle et la pression est choisie dans la gamme allant de 800 à 960 mbar.

La température de la réaction est généralement la température habituelle, comprise entre 0°C et 120°C ou égale à ces valeurs, et de préférence comprise entre environ 40°C et environ 90°C.

De préférence, la réaction est effectuée dans des conditions anhydres.

Un des avantages du procédé selon l'invention est que la durée de réaction est raccourcie et peut même être diminuée de moitié par rapport à celle de l'art antérieur, en particulier dans des solvants tels que les esters. Ces derniers solvants étant de plus moins chers, la mise en oeuvre du procédé selon l'invention entraîne de ce fait une réelle économie.

Lorsque la réaction est terminée, les produits sont isolés selon les procédures classiques. Le phosgène et le solvant sont généralement éliminés par distillation sous pression très faible.

Le rendement obtenu en NCA, après cristallisation, est nettement amélioré et souvent supérieur ou égal à 90 %. Le taux de chlore hydrolysable est inférieur à 0,05 %.

En conséquence, les NCA préparés selon le procédé de l'invention pourront être utilisés dans les nombreuses applications pour lesquelles des produits très purs sont exigés et notamment pour l'obtention de produits pharmaceutiques.

Les exemples qui suivent illustrent l'invention sans toutefois la limiter.

### EXEMPLE 1 :

### Préparation du N-carboxyanhydride de l'ester γ-benzylique de l'acide glutamique (H-Glu(OBzl)-NCA).

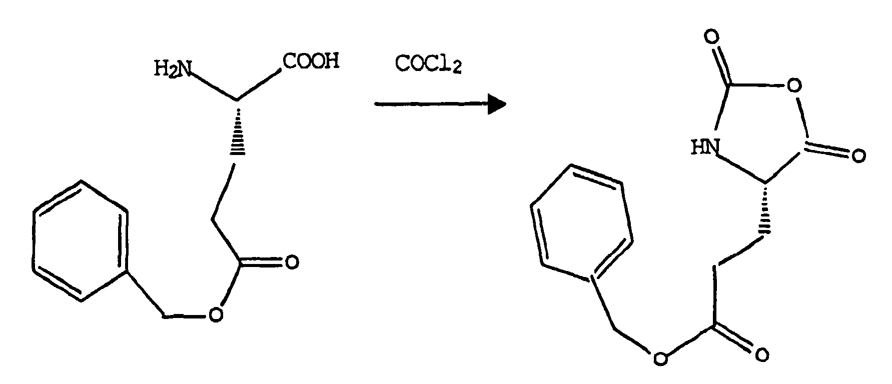

100 g (0,42 mol) de H-Glu(OBzl)-OH sont mis en suspension dans 885 ml d'acétate d'éthyle. On refroidit la suspension à 5°C puis on introduit dans celle-ci 90 g (0,91 mol, 2,16 éq) de phosgène gazeux en 1 heure 30, à une température de 10°C.

On porte la température du milieu réactionnel à 60°C puis on met le milieu réactionnel sous pression réduite (850-950 mbar) et on laisse en palier 3 heures à une température de masse de 60°C. Le milieu devient limpide au bout d'une heure de palier.

On effectue ensuite une distillation aux environs de 13 mbar pour séparer 600 ml d'un mélange acétate d'éthyle et de phosgène. On ajoute à chaud dans le milieu restant 600 ml d'heptane industriel et on le refroidit à 0°C pendant 1 heure. Le produit qui a cristallisé est filtré et lavé à l'heptane industriel.

Après séchage, on obtient 106 g (rendement : 95,5 %) de H-Glu(OBzl)-NCA dont le taux de chlore hydrolysable est inférieur à 0,05 %.

### EXEMPLE COMPARATIF 1 :

### Préparation du N-carboxyanhydride de l'ester γ-benzylique de l'acide glutamique (H-Glu(OBzl)-NCA).

100 g (0,42 mol) de H-Glu(OBzl)-OH sont mis en suspension dans 885 ml d'acétate d'éthyle. On refroidit la suspension à 5°C puis on y introduit 90 g (0,91 mol, 2,16 éq) de phosgène gazeux.

On chauffe le milieu réactionnel à 60°C. La réaction se déroule lentement. Le milieu réactionnel doit être laissé en palier 6 heures à cette température au lieu de 3 heures dans l'exemple précédent.

On distille ensuite comme précédemment pour séparer 600 ml d'un mélange acétate d'éthyle et de phosgène. On ajoute dans le milieu restant, à chaud, 600 ml d'heptane industriel et on le refroidit à - 10°C pendant 2 heures. Le produit qui a cristallisé est filtré et lavé à l'heptane industriel.

Après séchage, on obtient 88 g de H-Glu(OBzl)-NCA, dont le taux de chlore hydrolysable est de 0,13 %. Le rendement est de 74,6 % seulement.

### EXEMPLE 2 :

### Préparation du N-carboxyanhydride de la N-(1-éthoxycarbonyl-3-phénylpropyl)alanine (EPAL-NCA).

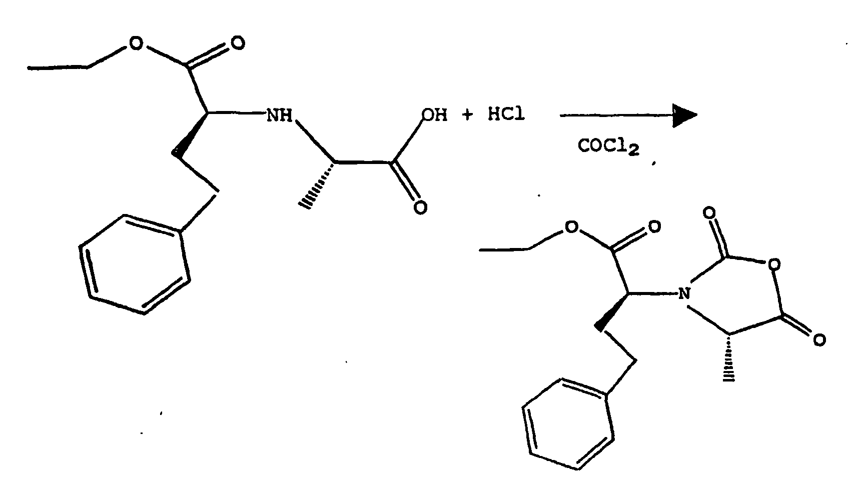

Dans un réacteur thermostaté de 1 litre préalablement inerté à l'azote, on introduit 350 ml d'acétate d'éthyle anhydre puis 42 g (0,15 mol, 1 équivalent) de N-(1-éthoxycarbonyl-3-phénylpropyl) alanine (EPAL). Dans la suspension obtenue agitée mécaniquement, on introduit alors 6 g (0,165 mol, 1,1 équivalent/EPAL) d'acide chlorhydrique gazeux anhydre, en 15 minutes à une température comprise entre 10°C et 28°C pour former le chlorhydrate d'EPAL.

On ajoute ensuite dans le milieu réactionnel 30 g (0,3 mol, 2 équivalents/EPAL) de phosgène gazeux en une heure. On chauffe ensuite le milieu à 60°C et on diminue la pression jusqu'aux environs de 800 mbar pour être au reflux de l'acétate d'éthyle. On maintient ces conditions pendant 3 heures. On constate alors, par analyse HPLC, qu'il n'y a plus d'EPAL dans le milieu réactionnel.

On élimine l'excès de phosgène, l'acide chlorhydrique restant et on sépare l'acétate d'éthyle en baissant la pression jusqu'aux environs de 13 mbar (1,3 kPa).

On ajoute ensuite 200 ml d'éther diisopropylique dans le milieu réactionnel concentré et on le refroidit à 0°-5°C. L'EPAL-NCA cristallise. On le recueille par filtration sous atmosphère d'azote.

Après séchage sous vide à la température de 20°-25°C, on obtient 41,2 g d'EPAL-NCA (solide blanc) de pureté supérieure à 99,7 % déterminée par HPLC et dont le taux de chlore hydrolysable est inférieur à 0,05 %. Le rendement est de 90 %.

### EXEMPLE 2 COMPARATIF :

### Préparation du N-carboxyanhydride de la N-(1-éthoxycarbonyl-3-phénylpropylalanine) (EPAL-NCA).

On utilise les mêmes quantités de composés que dans l'exemple précédent et on opère de la même façon et dans les mêmes conditions à l'exception de la pression de la réaction qui n'est pas diminuée et qui reste la pression atmosphérique normale.

Après huit heures de réaction à 60°C, 3,73 % en poids d'EPAL n'ayant pas réagi reste encore dans le milieu réactionnel et on n'observe plus aucune transformation.

### EXEMPLE 3 :

### Préparation du N-carboxyanhydride de l'alanine (H-Ala-NCA).

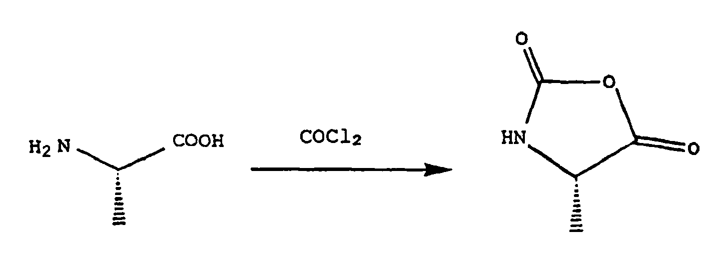

25 g (0,285 mol) d'alanine (H-Ala-OH) sont mis en suspension dans 220 ml d'acétate d'éthyle. On introduit ensuite dans la suspension 70,5 g (0,71 mol, 2,5 éq) de phosgène gazeux en 1 heure 30 à une température de 10°C.

On chauffe le milieu réactionnel à 55°C puis on le met sous pression réduite (850-950 mbar) et on le laisse ainsi en palier 6 heures à une température de masse de 55°C. Il devient limpide au bout de 3 heures de palier.

On distille ensuite sous une pression très faible pour séparer 200 ml d'un mélange acétate d'éthyle et de phosgène.

On ajoute alors à chaud, sur le milieu restant, 80 ml de toluène et l'on effectue une autre distillation pour séparer 78 g d'un mélange acétate d'éthyle et de toluène. On refroidit ensuite à 0°C le milieu restant pendant 1 heure. Le produit qui a cristallisé est filtré et lavé par 39 g de toluène froid.

Après séchage, on obtient 19,4 g (rendement : 59,2 %) de H-Ala-NCA dont le taux de chlore hydrolysable est inférieur à 0,05 %.

## Revendications

1. Procédé de préparation des N-carboxyanhydrides par réaction de l'α-, β- ou γ-amino-acide correspondant ou d'un de ses sels obtenus par réaction du groupe amino avec des acides, avec le phosgène, le diphosgène et/ou le triphosgène, dans un milieu solvant, **caractérisé en ce que** le milieu solvant est l'acétate d'éthyle et au moins une partie de la réaction est effectuée sous une pression choisie dans la gamme allant de 800 à 960 mbar.

2. Procédé selon la revendication 1, **caractérisé en ce que** la réaction est effectuée avec le phosgène.

3. Procédé selon l'une quelconque des revendications précédentes, **caractérisé en ce que** lorsque le radical de l'amino-acide comporte des groupes réactifs autres que le groupe acide et le groupe amino formant l'anhydride, ils sont protégés.

4. Procédé selon l'une quelconque des revendications précédentes, **caractérisé en ce que** l'amino-acide de départ est choisi dans le groupe comprenant la leucine, l'alanine, la N-trifluoroacétyl-lysiné, l'ester γ-benzylique et l'ester γ-méthylique de l'acide glutamique, la N-(1-éthoxycarbonyl-3-phénylpropyl)alanine et leurs sels.

5. Procédé selon l'une quelconque des revendications précédentes, **caractérisé en ce que** le sel de l'amino-acide est un sulfate, un acétate, un toluènesulfonate ou un méthanesulfonate.

6. Procédé selon l'une quelconque des revendications précédentes, **caractérisé en ce que** le sel de l'amino-acide est un halohydrate.

7. Procédé selon l'une quelconque des revendications précédentes, **caractérisé en ce que** la réaction est effectuée à une température comprise entre 0°C et 120°C.

## Claims

1. Process for the preparation of N-carboxyanhydrides by reaction of the corresponding α-, β- or γ-amino acid or one of its salts, obtained by reaction of the amino group with acids, with phosgene, diphosgene and/or triphosgene in a solvent medium, **characterized in that** the solvent medium is ethyl acetate and at least a portion of the reaction is carried out under a pressure chosen within the range from 800 to 960 mbar.

2. Process according to Claim 1, **characterized in that** the reaction is carried out with phosgene.

3. Process according to either one of the preceding claims, **characterized in that**, when the radical of the amino acid comprises reactive groups other than the acid group and the amino group forming the anhydride, they are protected.

4. Process according to any one of the preceding claims, **characterized in that** the starting amino acid is chosen from the group comprising leucine, alanine, N-(trifluoroacetyl)lysine, the γ-benzyl ester and the γ-methyl ester of glutamic acid, N-(1-ethoxycarbonyl-3-phenylpropyl)alanine and their salts.

5. Process according to any one of the preceding claims, **characterized in that** the amino acid salt is a sulphate, an acetate, a toluenesulphonate or a methanesulphonate.

6. Process according to any one of the preceding claims, **characterized in that** the amino acid salt is a hydrohalide.

7. Process according to any one of the preceding claims, **characterized in that** the reaction is carried out at a temperature of between 0°C and 120°C.

## Patentansprüche

1. Verfahren zur Herstellung von N-Carboxyanhydriden durch Umsetzung der entsprechenden α-, β- oder γ-Aminosäure oder eines ihrer Salze, die durch Umsetzung der Aminogruppe mit Säuren erhalten werden, mit Phosgen, Diphosgen und/oder Triphosgen in einem Lösungsmitlelmedium, **dadurch gekennzeichnet, dass** das Lösungsmittelmedium Ethylacetat ist und mindestens ein Teil der Umsetzung unter einem Druck bewirkt wird, der aus dem Bereich von 800 bis 960 mbar ausgewählt ist.

2. Verfahren nach Anspruch 1, **dadurch gekennzeichnet, dass** die Umsetzung mit Phosgen bewirkt wird.

3. Verfahren nach irgendeinem der vorangehenden Ansprüche, **dadurch gekennzeichnet, dass**, wenn der Aminosäure-Rest andere reaktive Gruppen umfasst als die Säuregruppe und die Aminogruppe, die das Anhydrid bilden, diese geschützt werden.

4. Verfahren nach irgendeinem der vorangehenden Ansprüche, **dadurch gekennzeichnet, dass** die Ausgangs-Aminosäure ausgewählt ist aus der Gruppe umfassend Leucin, Alanin, N-Trifluoracetyllysin, Glutaminsäure-γbenzylester und -γ-methylester, N-(1-Ethoxycarbonyl-3-phenylpropyl)alanin und deren Salze.

5. Verfahren nach irgendeinem der vorangehenden Ansprüche, **dadurch gekennzeichnet, dass** das Salz der Aminosäure ein Sulfat, ein Acetat, ein Toluolsulfonat oder ein Methansulfonat ist.

6. Verfahren nach irgendeinem der vorangehenden Ansprüche, **dadurch gekennzeichnet, dass** das Salz der Aminosäure ein Hydrohalogenid ist.

7. Verfahren nach irgendeinem der vorangehenden Ansprüche, **dadurch gekennzeichnet, dass** die Umsetzung bei einer Temperatur zwischen 0°C und 120°C einschließlich bewirkt wird.
